# EUROPEAN PATENT SPECIFICATION

(11) **EP 0 744 917 B1**
(45) Date of publication and mention of the grant of the patent: **08.07.1998**
(21) Application number: 95908323.9
(22) Date of filing: 15.02.1995
(51) Int. Cl.: A61B 17/16, A61F 2/46

(54) **ARTICLE TO BE USED IN SURGERY AND MANIPULATING TOOL**
GEGENSTAND UND WERKZEUG ZUM GEBRAUCH IN DER CHIRURGIE
ARTICLE DESTINE A ETRE UTILISE EN CHIRURGIE ET OUTIL SERVANT A LE MANIER

(30) Priority: 19.02.1994 GB 9403219
(43) Date of publication of application: 04.12.1996
(73) Proprietor: DEPUY INTERNATIONAL LIMITED, Beeston, Leeds LS11 8DT (GB)
(72) Inventor: COHEN, Andrew, Beal Nr. Goole DN14 0SL (GB); DAVIES, Hugh, Leeds LS11 6HT (GB); ISAAC, Graham, Huddersfield HD7 7BJ (GB); McSHANE, Gerry, Ossett WF5 0NE (GB)
(74) Representative: Belcher, Simon James
(86) International application number: GB9500311
(87) International publication number: WO9522286

(56) References cited:
- US-A- 4 601 289
- US-A- 4 990 149
- US-A- 5 089 003

## Description

This invention relates to an assembly of an article to be used in surgery and a tool for manipulating the article.

It is important to be able to control articles used in surgery, whether the articles be tools or for implantation. For example, considerable force can be required to be exerted on tools used in orthopaedic surgery, for example to cut or to shape bone. It is vital that the force be exerted in a controlled manner, to ensure that the operation involving the tool be carried out accurately.

It is known to shape a recess in the medulla of a femur to receive subsequently the femoral component of a hip prostheses. The medulla is shaped using a broach, which is shaped appropriately for the desired shape of recess, and which has an appropriately roughened outer surface to abrade the femur. The recess is shaped by moving the broach in to and out of the cavity. In order to provide an appropriately sized recess in the femur, it is common to use a number of broaches, of increasing size. A small broach is used initially, followed by broaches which increase in size until the recess in the femur has reached the desired size.

Articles that are to be used in surgery, such as broaches or other tools, or implants, can be provided with handles by which they can be controlled during the surgery. However, it can be preferred in many situations for an article to be capable of being attached releasably to a handle. This can be particularly desirable in the case of, for example, the broach arrangement described above, when a succession of broaches are to be used. In this situation, it can be desirable for each of the succession of broaches to be attached releasably to a handle, so that inventory is minimised.

US-4601289 discloses an assembly for use in hip implant surgery which includes a handle assembly which locks onto a trial prosthesis or rasp. The handle assembly has a first recess for partially receiving a post on the prosthesis or rasp and a pivotable lever with an opposing recess for to be fitted over the part of the post not received in the recess when a screw is tightened.

US-5089003 discloses a rasp tool which includes a handle member with a releasable locking mechanism for selectively coupling the handle to a cutter member. The locking mechanism includes a locking post on the cutter member and a corresponding bore in the handle member. A spring biassed locking key is disposed within a guide bore that intersects the post receiving bore so that the locking key extends partially into the bore, to engage the locking post when inserted therein.

The present invention provides an assembly of an article to be used in surgery and a tool (such as a handle) for manipulating the article, the article and tool being connected to one another by means of pairs of mating lugs and recesses.

Accordingly, the invention provides an assembly of an article to be used in surgery and a tool for manipulating the article, the article and the tool having two pairs of mating surfaces which are exposed externally of the article and tool before they are mated with one another for use, and arranged so that the pairs of surfaces abut when the article and the tool are mated with one another, the said mating surfaces bearing respective lugs and recesses in two mating pairs of which the lugs can be received in their respective recesses with a snug fit to connect the article and the tool to one another, the lugs and recesses being arranged so that the lug and recess of one of the pairs are not parallel to those of the other pair, at least one of the said lugs being provided on the tool in such a way that that lug can be moved between a retracted position and an extended position characterised in that its extension beyond its mating surface is increased when the tool is viewed externally in profile, the connection between the article and the tool being made when the lug is in that extended position.

The assembly of the present invention has the advantage of providing a conveniently and quickly formed connection between the article and the tool. This is important. The user of the article and tool (generally a surgeon) manipulates such an assembly in difficulty conditions, wearing one or two pairs of gloves. Furthermore, his gloves and the components of the assembly are likely to be coated with body fluids, making manipulation of the components difficult. The assembly of the present invention facilitates simple interconnection of an article to be used in surgery and an appropriate manipulation tool, by engaging one of the lugs in its recess, and then moving the other of the lugs from its retracted position to its extended position so that it engages its recess. This arrangement can be contrasted with existing interconnection arrangements which use threaded connectors and are difficult to operate.

The ease of manipulation that is made possible by the assembly of the present invention has significant advantages for a patient. Firstly, there can be reductions in the time taken to perform surgery because of the ease with which tools can be selected and prepared for use, minimising complications arising from anaesthesia. Secondly, a surgeon will be more inclined to change tools where necessary, and will not be put off making this change because of difficulties in manipulating the tools.

A further significant advantage of the assembly of the invention is that the space that is occupied by the tool when it engages the article can be arranged to be small. This enables the assembly of the interconnected article and tool to be used in applications where space is restricted. This can make it possible to connect a tool and an article in such a way that they are appropriately aligned for use of the article, especially with axes of the article and the tool substantially parallel to or aligned with one another, or both. This has the significant advantage of facilitating greater control over the article when it is in use, for example when the article is a broach being used to shape a recess within the medulla of a femur.

Preferably, the angle between the axes of the pins and/or recesses on one of the article and tool is at least about 30°, more preferably at least about 50°, especially at least about 70°. It is particularly preferred that the axes of the pins and/or recesses are approximately perpendicular to one another. This facilitates the formation of a secure connection between the article and tool.

Preferably, the tool has a longitudinal body portion, and the axis of one of the pins and/or recesses that is provided on the tool is substantially parallel to the axis of the body portion. This arrangement facilitates location of that pin in its corresponding recess, by relative movement of the tool towards the article along the axis of the tool.

The assembly can include other features by which the connection between the tool and the article can be stabilised further. For example, more than two pairs of mating lugs and recesses can be provided on the tool and article. Alternatively, or in addition, the article and the tool may have respective surfaces which abut one another when the article and tool are connected to one another by means of the lugs and recesses. It can be preferred for such surfaces on the article and tool to be arranged so that they provide surface to surface contact between the components (rather than point or line contact).

The recesses in which the lugs fit need not be closed around their entire periphery. It is, however, preferred that they be closed in this way.

It can be appropriate for some applications for the moveable lug to be mounted for retraction in to, and extension out of, the other lug. In this event, the recesses will be similarly arranged with that for the moveable lug communicating with that for the fixed lug.

Preferably, both of the lugs are provided on the tool, with both of the corresponding recesses provided on the article.

Preferably the moveable lug that is provided on the tool is mounted for pivotal movement between its retracted and extended positions. For example, the tool can include a pivotal member on which the moveable lug is provided. The pivotal member can be mounted to pivot around an appropriate fulcrum in or on the tool. An actuator can be provided by which the pivotal member can be moved to move the pin between its retracted and extended position.

Preferably, the pivotal member on which the moveable lug is provided is located at least partially within a housing within a tool. It is particularly preferred that all of the pivotal member be located within the housing, with just the moveable lug extending from the housing when in its extended position.

Preferably, the actuator for the pivotal member comprises a rotatable member which has a camming surface which engages the pivotal members. The pivotal member can be biased resiliently towards the retracted or extended position of the moveable lug, especially towards the extended position.

The article of the assembly can have an abrading surface, especially so that it can be used as a broach for shaping a recess in a femur. The article can be intended for other uses in surgery, for example to apply force to a shapeable material, for example a cement or filler that might be used in orthopaedic surgery.

The article of the assembly can itself be a component of an osteoprosthetic implant. For example, the article might be the femoral component of a prosthetic hip. It might be another component of a prosthetic hip, or a component of another prosthetic joint such as a prosthetic knee or a prosthetic shoulder.

The invention also provides components of the assembly discussed above. The invention further provides a kit which comprises an assembly of a first article to be used in surgery and a tool for manipulating the article, as discussed above, and at least one further article which can be connected to the tool in place of the said first article.

The present invention will now be described, by way of example only, with reference to the accompanying drawings, in which:
Figure 1 is a side view of the tool of the assembly of the invention;
Figure 2 is a front view of the handle component of the tool shown in Figure 1;
Figure 3 is a view from below of the actuator of the tool shown in Figure 1;
Figure 4 is a side view, partially in section, of a broach to which the tool shown in Figure 1 can be connected;
Figure 5 is a front view of the broach shown in Figure 4.

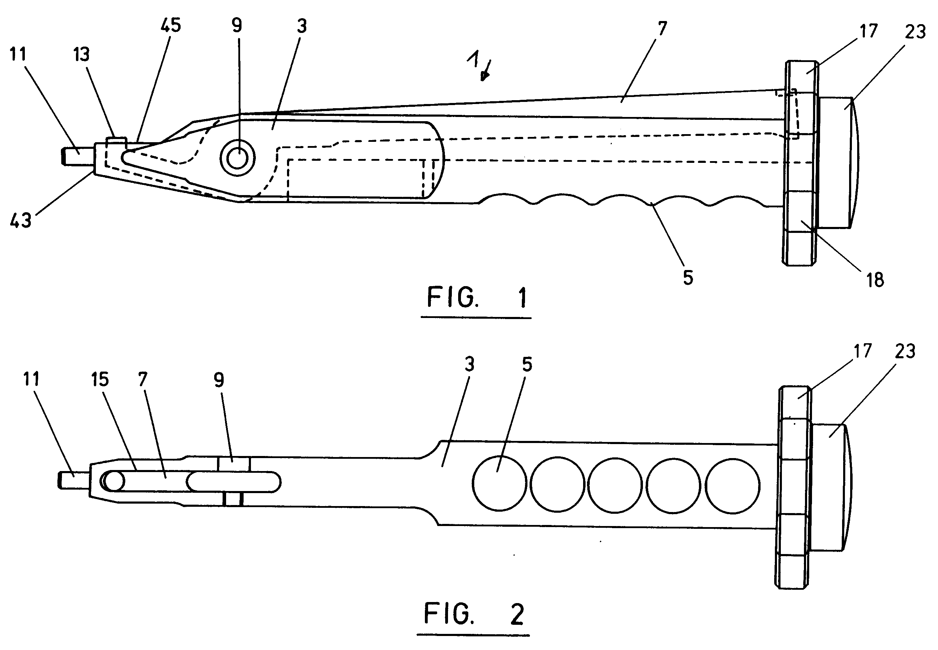

Figures 1 and 2 shows a tool 1 which can be used to manipulate an article to be used in surgery such as a broach or a prosthetic implant. The tool comprises a handle 3 with appropriate formations 5 to enable it to be grasped during use. The handle is hollow. It contains a pivotal member 7 arranged to pivot about a fulcrum 9.

The tool 1 has a fixed lug 11 extending from its end, the axis of the lug being parallel to and aligned with the axis of the tool. A moveable lug 13 is provided on the pivotal member 7. The moveable lug 13 can move between retracted and extended positions, through a slot 15 in the handle 3.

The tool 1 includes an actuating member 17. The member 17 is mounted for rotation on one end of the handle 3. It has notches 18 cut into its periphery to facilitate grip.

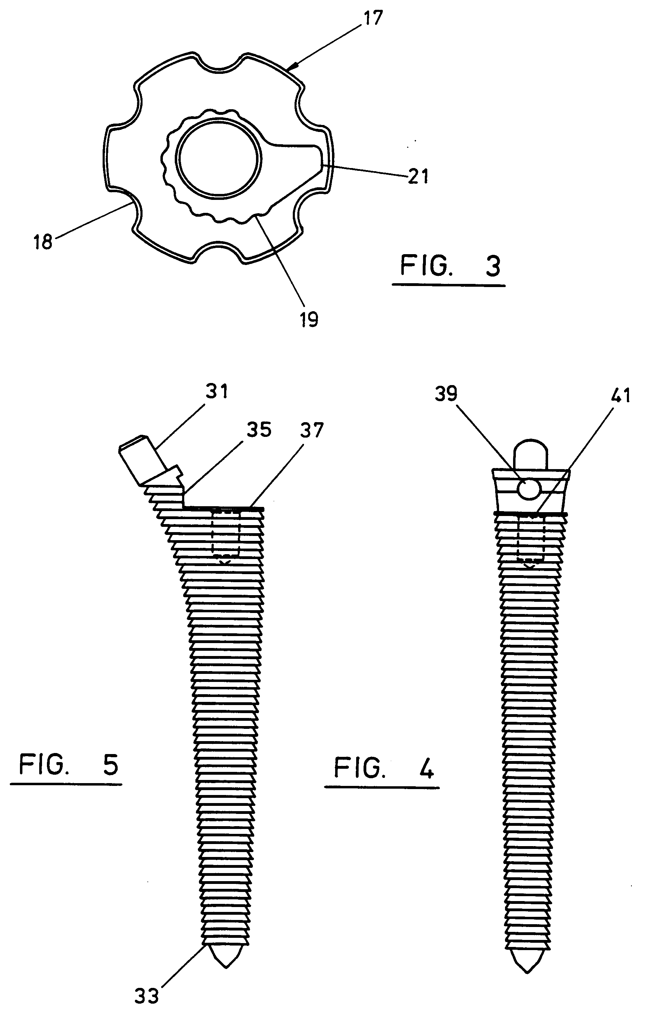

Figure 3 shows the actuator member 17 from below. The member has a raised profiled cam surface, arranged to engage the free end of the pivotal member 7 as the actuator member 17 is rotated about the axis of the handle. As the member is rotated, the free end of the pivotal member is driven along the camming surface 19, towards the free end 21 of the camming surface 19. As this happens, the pivotal member is driven away from the centre line of the handle, and the moveable lug 13 is caused to move from its retracted position to the extended position, in which it is received in a corresponding recess in the article to be connected to the tool.

A fixing cap 23 retains the actuator member in place on the end of the handle 3.

Referring to Figures 4 and 5, the broach 31 comprises an elongate body that has an approximately circular cross-section over a large part of its length, especially towards its free end 33. The outer surface of the broach is provided by a plurality of individual teeth, by which the surface of a recess in a femur can be abraded.

At its upper end 35, the broach 31 has a cut-out shoulder 37 at which the connection is formed to the tool 1. There is a medially extending recess 39, and a downwardly extending recess 41. The recesses are cut into planar surfaces which define the shoulder, which abut corresponding planar surfaces 43, 45 on the tool, from which the lugs 11, 13 extend.

When the end of the tool is received in a recess in the article, to the extent that the opening through which the moveable lug extends from the tool is located in the recess, the end of the tool can itself be considered as the fixed lug (optionally with a discrete lug extending from it), and the recess for the movable lug will communicate with the recess in which the end of the tool is received.

The tool 1 can be connected to the broach by first locating the fixed lug 11 in the downwardly extending recess 41, while the moveable lug 13 is in its retracted position within the handle 3 in the tool 1. The actuator member 17 can then be rotated to cause the moveable lug 13 to extend from the slot 15 in the handle 3, to be received in the laterally extending recess 39 in the broach 31. The recesses and lugs are so arranged on the tool and the broach that the longitudinal axes of the broach and the tool are parallel to and aligned with one another. This enables the broach to be used reliably to abrade bone in a recess, with the force being applied to the broach reliably along its axis.

## Claims

1. An assembly of an article (31) to be used in surgery and a tool (1) for manipulating the article, the article and the tool having two pairs of mating surfaces (43, 45; 37) which are exposed externally of the article and tool before they are mated with one another for use, and arranged so that the pairs of surfaces abut when the article and the tool are mated with one another, the said mating surfaces bearing respective lugs and recesses (11, 13; 39, 41) in two mating pairs of which the lugs can be received in their respective recesses with a snug fit to connect the article and the tool to one another, the lugs and recesses being arranged so that the lug and recess of one of the pairs are not parallel to those of the other pair, at least one of the said lugs being provided on the tool in such a way that that lug can be moved between a retracted position and an extended position characterised in that its extension beyond its mating surface is increased when the tool is viewed externally in profile, the connection between the article and the tool being made when the lug is in that extended position.

2. An assembly as claimed in claim 1, in which the angle between the axes of the lugs and/or recesses (11, 13; 39, 41) on one of the article (31) and the tool (1) is at least 30°.

3. An assembly as claimed in claim 2, in which the said angle is at least 70°.

4. An assembly as claimed in any one of claims 1 to 3, in which the tool (1) has a longitudinal body portion, and the axis of one of lugs and/or recesses (11, 13) that is provided on the tool is substantially parallel to the axis of the body portion.

5. An assembly as claimed in any one of claims 1 to 4, in which two lugs are provided on the tool (1) and two corresponding recesses are provided on the article (31).

6. An assembly as claimed in any one of claims 1 to 5, in which the movable lug (13) that is provided on the tool (1) is mounted for pivotal movement between its retracted and extended positions.

7. An assembly as claimed in claim 6, in which the tool (1) includes a pivotal member (7) on which the movable lug (13) is provided, and an actuator (17) by which the pivotal member (7) can be moved to move the lug between its retracted and extended positions.

8. An assembly as claimed in claim 7, in which the pivotal member (7) is located at least partially within a housing (3) within the tool.

9. An assembly as claimed in claim 7 or claim 8, in which the actuator (17) comprises a rotatable member, which has a camming surface (19) which engages the pivotal member.

10. An assembly as claimed in any one of claims 1 to 9 in which the article (31) has an abrading surface.

11. An assembly as claimed in claim 10, in which the article (31) is a broach.

12. An assembly as claimed in any one of claims 1 to 9, in which the article is a component of an osteoprosthetic implant.

13. An assembly as claimed in any one of claims 1 to 12, in which the mating surfaces are substantially planar.

14. A kit which comprises an assembly of a first article (31) to be used in surgery and a tool (1) for manipulating the article, as claimed in any one of claims 1 to 13, and at least one further article which can be connected to the tool in place of the said first article.

## Patentansprüche

1. Anordnung aus einem Gegenstand (31) zum Gebrauch in der Chirurgie und einem Werkzeug (1), zur Bedienung des Gegenstands, bei welcher der Gegenstand und das Werkzeug zwei Paare zueinander passender Flächen (43, 45; 35, 37) aufweisen, die, bevor sie bei der Benutzung zusammengebracht sind, offen an der Außenseite des Gegenstandes und des Werkzeuges zugänglich sind und so angeordnet sind, daß die Flächenpaare aneinanderstoßen, wenn der Gegenstand und das Werkzeug zusammengebracht sind, wobei die genannten zueinander passenden Flächen jeweils Nasen und Vertiefungen (11, 13; 39, 41) in zwei zueinanderpassenden Paaren aufweisen, in denen die Nasen in ihren jeweiligen Vertiefungen mit einem Paßsitz aufgenommen werden können, wodurch der Gegenstand und das Werkzeug miteinander verbunden werden, wobei die Nasen und die Vertiefungen so eingerichtet und angeordnet sind, daß die Nase und die Vertiefung des einen Paares nicht parallel zu denjenigen des anderen Paares sind, und wenigstens eine der genannten Nasen so an dem Werkzeug vorgesehen ist, daß diese Nase zwischen einer zurückgezogenen und einer ausgefahrenen Position bewegt werden kann,
dadurch gekennzeichnet, daß
ihre Erstreckung über ihre passende Fläche hinaus vergrößert ist, wenn das Werkzeug im Profil von außen betrachtet wird, wobei die Verbindung zwischen dem Gegenstand und dem Werkzeug dann erfolgt, wenn die Nase in dieser ausgefahrenen Position ist.

2. Anordnung nach Anspruch 1, bei welcher der Winkel zwischen den Achsen der Nasen und/oder der Vertiefungen (11, 13; 39, 41) auf dem Gegenstand (31) bzw. dem Werkzeug (1) wenigstens 30° beträgt.

3. Anordnung nach Anspruch 2, bei welcher genannter Winkel wenigstens 70° beträgt.

4. Anordnung nach einem der Ansprüche 1 bis 3, bei welcher das Werkzeug einen longitudinalen Körperabschnitt aufweist, und die Achse einer der Nasen und/oder einer der Vertiefungen (11, 13), die an dem Werkzeug angebracht sind, im wesentlichen parallel zu der Achse des Körperabschnitts ist.

5. Anordnung nach einem der Ansprüche 1 bis 4, bei welcher zwei Nasen an dem Werkzeug (1) und zwei entsprechende Vertiefungen an dem Gegenstand (31) vorgesehen sind.

6. Anordnung nach einem der Ansprüche 1 bis 5, bei welcher die bewegliche Nase (13) die an dem Werkzeug (1) vorgesehen ist, schwenkbar zwischen ihrer zurückgezogenen und ihrer ausgefahrenen Position anmontiert ist.

7. Anordnung nach Anspruch 6, bei welcher das Werkzeug (1) ein Schwenkglied (7), auf welchem die bewegliche Nase (13) vorgesehen ist, und eine Betätigungseinrichtung (17) aufweist, mit der das Schwenkglied (7) so bewegt werden kann, daß die Nase zwischen ihrer versenkten und ihrer ausgefahrenen Position bewegt wird.

8. Anordnung nach Anspruch 7, bei welcher das Schwenkglied (7) sich wenigstens teilweise innerhalb eines Gehäuses (3) innerhalb des Werkzeugs befindet.

9. Anordnung nach Anspruch 7 oder Anspruch 8, bei welcher die Betätigungseinrichtung (17) ein Drehglied enthält, welches eine Nockenoberfläche (19) aufweist, die an dem Schwenkglied angreift.

10. Anordnung nach einem der Ansprüche 1 bis 9, bei welcher der Gegenstand (31) eine abradierende Oberfläche aufweist.

11. Anordnung nach Anspruch 10, bei welcher der Gegenstand (31) ein Dorn ist.

12. Anordnung nach einem der Ansprüche 1 bis 9, bei welcher der Gegenstand Teil eines osteoprothetischen Implantats ist.

13. Anordnung nach einem der Ansprüche 1 bis 12, bei welcher die zueinander passenden Flächen im wesentlichen eben sind.

14. Ein Bausatz, der aus einer Anordnung eines ersten Gegenstandes (31) zum Gebrauch in der Chirurgie und einem Werkzeug (1) zur Bedienung des Gegenstandes, nach einem der Ansprüche 1 bis 13, und wenigstens einem weiteren Gegenstand, der mit dem Werkzeug statt des genannten ersten Gegenstands verbunden werden kann, besteht.

## Revendications

1. Assemblage d'un article (31) destiné à être utilisé en chirurgie et d'un outil (1) servant à manier l'article, l'article et l'outil comportant deux paires de surface d'accouplement (43, 45 ; 37) qui sont exposées à l'extérieur de l'article et de l'outil avant d'être accouplées les unes aux autres pour utilisation, et agencés de façon que les paires de surface viennent en butée lorsque l'article et l'outil sont accouplés l'un à l'autre, lesdites surfaces d'accouplement, portant des tenons et des évidements respectifs (11, 13 ; 39, 41) en deux paires appariées, parmi lesquelles les tenons peuvent être reçus dans leurs évidements respectifs en ajustement étroit afin de relier l'article et l'outil l'un à l'autre, les tenons et évidements étant agencés de façon que le tenon et l'évidement de l'une des paires ne soit pas parallèle à ceux de l'autre paire, au moins l'un desdits tenons étant agencé sur l'outil de telle façon que ce tenon puisse être déplacé entre une position rentrée et une position sortie, caractérisé en ce que son extension au-delà de sa surface d'accouplement est augmentée lorsque l'on observe l'outil extérieurement en profil, la liaison entre l'article et l'outil étant réalisée lorsque le tenon se trouve dans cette position sortie.

2. Assemblage selon la revendication 1, dans lequel l'angle entre les axes des tenons et/ou évidements (11, 13 ; 39, 41) sur l'un de l'article (31) et de l'outil (1) est au moins de 30°.

3. Assemblage selon la revendication 2, dans lequel ledit angle est d'au moins 70°.

4. Assemblage selon l'une quelconque des revendications 1 à 3, dans lequel l'outil (1) comporte une partie de corps longitudinale, et l'axe de l'un des tenons et/ou évidemment (11, 13) qui est formé sur l'outil est pratiquement parallèle à l'axe de la partie de corps.

5. Assemblage selon l'une quelconque des revendications 1 à 4, dans lequel deux tenons sont formés sur l'outil (1) et deux évidements correspondants sont formés sur l'article (31).

6. Assemblage selon l'une quelconque des revendications 1 à 5, dans lequel le tenon mobile (13) qui est disposé sur l'outil (1) est monté en vue d'un mouvement pivotant entre ses positions rentrée et sortie.

7. Assemblage selon la revendication 6, dans lequel l'outil (1) comprend un élément pivotant (7) sur lequel le tenon mobile (13) est formé, et un actionneur (17) par lequel l'élément pivotant (7) peut être déplacé afin de déplacer le tenon entre ses positions rentrée et sortie.

8. Assemblage selon la revendication 7, dans lequel l'élément pivotant (7) est situé au moins en partie à l'intérieur d'un logement (3) dans l'outil.

9. Assemblage selon la revendication 7 ou la revendication 8, dans lequel l'actionneur (17) comprend un élément rotatif, qui comporte une surface de came (19) qui attaque l'élément pivotant.

10. Assemblage selon l'une quelconque des revendications 1 à 9, dans lequel l'article (31) comporte une surface abrasive.

11. Assemblage selon la revendication 10, dans lequel l'article (31) est une broche.

12. Assemblage selon l'une quelconque des revendications 1 à 9, dans lequel l'article est un composant d'un implant d'ostéoprothèse.

13. Assemblage l'une quelconque des revendications 1 à 12, dans lequel les surfaces d'accouplement sont pratiquement planes.

14. Nécessaire qui comprend un assemblage d'un premier article (31) destiné à être utilisé en chirurgie et d'un outil (1) permettant de manier l'article, selon l'une quelconque des revendications 1 à 13, ainsi qu'au moins un article supplémentaire qui peut être fixé à l'outil au lieu dudit premier article.
